# EUROPEAN PATENT APPLICATION

(11) **EP 4 439 592 A1**
(43) Date of publication of application: **02.10.2024**
(21) Application number: 24167426.6
(22) Date of filing: 28.03.2024
(51) Int. Cl.: H01F 1/00, G01N 33/543

(54) **BIOLOGICAL MATERIAL EXTRACTION MAGNETIC BEAD**

(30) Priority: 30.03.2023 JP 2023054844
(71) Applicant: Seiko Epson Corporation, Tokyo 160-8801 (JP)
(72) Inventor: TSUKADA, Kenta, Suwa-shi, 392-8502 (JP); HANAMURA, Masato, Suwa-shi, 392-8502 (JP); UCHIDA, Miki, Suwa-shi, 392-8502 (JP)
(74) Representative: Lewis Silkin LLP

(57) **Abstract**

A biological material extraction magnetic bead contains: a magnetic metal powder; an inorganic oxide layer that covers a particle surface of the magnetic metal powder and contains an inorganic oxide; a base layer that covers a surface of the inorganic oxide layer and contains a gold ion reducing agent or a catalyst for a gold ion reduction reaction; a gold layer that covers a surface of the base layer and contains gold; and an immobilization layer that is bonded to a surface of the gold layer via an Au-S bond and contains a compound having a ligand or a ligand binding site.

## Description

The present application is based on, and claims priority from JP Application Serial Number 2023-054844, filed March 30, 2023, the disclosure of which is hereby incorporated by reference herein in its entirety.

### BACKGROUND

### 1. Technical Field

The present disclosure relates to a biological material extraction magnetic bead.

### 2. Related Art

In recent years, in the fields of diagnosis and biological science in the medical field, there is an increasing demand for examination of a biological material. Among biological material examination techniques, a polymerase chain reaction (PCR) method is a method of extracting a nucleic acid such as deoxyribonucleic acid (DNA) or ribonucleic acid (RNA), specifically amplifying and detecting the nucleic acid. In such a process of examining the biological material, it is first necessary to extract a substance to be examined from a specimen. In the extraction of the biological material, for example, an extraction carrier having a binding ability for the biological material is used.

JP-A-2017-122728 discloses a ligand as a binding species having a binding ability to a cell surface receptor or a virus. It is also disclosed that a biomolecule is detected using a colloid holding the ligand. It is also disclosed that a self-assembled monolayer containing thiol is formed at a surface of a particle covered with gold, and the self-assembled monolayer presents a binding partner of the ligand.

JP-A-2017-122728 is an example of the related art.

In the extraction of the biological material, various physical and chemical loads are applied to the extraction carrier. When such loads are applied to the colloid holding the ligand disclosed in JP-A-2017-122728, the biological material bound to the extraction carrier may be detached or the extraction carrier may aggregate, and thus biological material extraction efficiency may decrease. The colloid disclosed in JP-A-2017-122728 has room for improvement in dispersibility in a dispersion medium. When the dispersibility is low, efficiency of capturing the biological material decreases, and as a result, the biological material extraction efficiency decreases.

Therefore, it is a problem to obtain a biological material extraction magnetic bead having favorable biological material extraction efficiency.

### SUMMARY

A biological material extraction magnetic bead according to an application example of the disclosure includes:
a magnetic metal powder;
an inorganic oxide layer that covers a particle surface of the magnetic metal powder and contains an inorganic oxide;
a base layer that covers a surface of the inorganic oxide layer and contains a gold ion reducing agent or a catalyst for a gold ion reduction reaction;
a gold layer that covers a surface of the base layer and contains gold; and
an immobilization layer that is bonded to a surface of the gold layer via an Au-S bond and contains a compound having a ligand or a ligand binding site.

### BRIEF DESCRIPTION OF THE DRAWINGS

FIG. 1 is a flowchart showing an example of a biological material extraction method.
FIG. 2 is a schematic diagram showing the biological material extraction method shown in FIG. 1.
FIG. 3 is a schematic diagram showing the biological material extraction method shown in FIG. 1.
FIG. 4 is a schematic diagram showing the biological material extraction method shown in FIG. 1.
FIG. 5 is a cross-sectional view showing a magnetic bead according to an embodiment.
FIG. 6 is a partially enlarged view of a coating film shown in FIG. 5.
FIG. 7 is a cross-sectional view showing a modification of the magnetic bead in FIG. 5.
FIG. 8 is a schematic diagram showing a state in which a compound having a functional site is bonded to a gold layer via an Au-S bond.

### DESCRIPTION OF EMBODIMENTS

Hereinafter, a preferred embodiment of a biological material extraction magnetic bead according to the disclosure will be described in detail with reference to the accompanying drawings.

The biological material extraction magnetic bead according to the embodiment is a particle group which adsorbs a biological material and which is used for magnetic separation in such a state. Magnetic separation is a technique of separating a solid phase and a liquid phase by applying an external magnetic field to a container in which a solid phase containing a biological material extraction magnetic bead and a liquid phase containing a dispersion medium are charged to magnetically attract the solid phase. In the following description, the biological material extraction magnetic bead may be simply referred to as a "magnetic bead".

The biological material refers to a substance such as a nucleic acid such as DNA or RNA, a protein, a saccharide, various cells such as a cancer cell, a peptide, a bacterium, and a virus. The nucleic acid may be present in a state of being contained in a biological sample such as a cell or biological tissue, a virus, or a bacterium. A biological material extraction method is a method of extracting such a biological material through each step of, for example, dissolution and adsorption, washing, and elution, and in such a process, the biological material can be purified and extracted by using the above-described magnetic separation.

### 1. Biological Material Extraction Method

Hereinafter, an example of a biological material extraction method using magnetic separation will be described. In the following description, a case where the biological material is a nucleic acid will be described as an example.

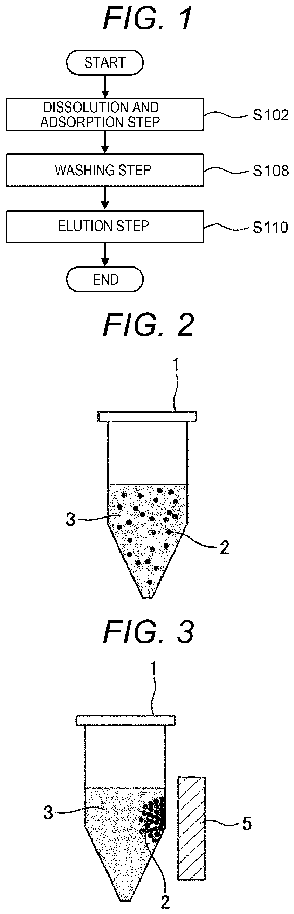

FIG. 1 is a flowchart showing an example of the biological material extraction method. FIGS. 2 to 4 are schematic diagrams showing the biological material extraction method shown in FIG. 1.

The biological material extraction method shown in FIG. 1 includes a dissolution and adsorption step S102, a washing step S108, and an elution step S110. Hereinafter, each step will be sequentially described.

### 1.1. Dissolution and Adsorption Step

In the dissolution and adsorption step S102, a specimen sample containing a nucleic acid, a liquid 3 containing a dissolution and adsorption liquid, and magnetic beads 2 are charged into a container 1 shown in FIG. 2. Then, contents in the container 1 are mixed. Accordingly, the magnetic beads 2 are dispersed in the liquid 3 in the container 1 as shown in FIG. 2. Since the nucleic acid is usually contained in a cell membrane and a nucleus, the nucleic acid is extracted by dissolving and removing the cell membrane and a so-called outer shell of the nucleus by a dissolution action of the dissolution and adsorption liquid. Thereafter, the nucleic acid is adsorbed to each magnetic bead 2 by an adsorption action of the dissolution and adsorption liquid.

As the dissolution and adsorption liquid, for example, a liquid containing a chaotropic substance is used. The chaotropic substance has a function of generating chaotropic ions in an aqueous solution, reducing an interaction between water molecules, and thereby destabilizing a structure, and thus contributes to adsorption of the nucleic acid to the magnetic bead 2.

Particularly, when RNA among nucleic acids is extracted, it is preferable to acidify the liquid 3 in the container 1 by adding an acid or the like. An RNA monomer contains ribose and thus is more soluble in a polar solvent than DNA. When RNA and DNA are separated using such a difference, the liquid 3 may be acidified by, for example, adding an acid after the outer shell is dissolved and removed. Thereafter, a non-polar solvent such as phenol or chloroform is added to the liquid 3. Accordingly, DNA migrates to the non-polar solvent whereas RNA remains in the polar solvent. As a result, RNA and DNA can be separated, and RNA can be extracted.

When RNA is extracted, a pH of the liquid 3 in the container 1 is preferably 5.0 or less, and more preferably 2.0 or more and 4.0 or less. Accordingly, an ionization equilibrium of a phosphate group contained in RNA is biased to a hydroxy group, and thus RNA is particularly easily dissolved in the polar solvent. Therefore, extraction efficiency of RNA can be further improved.

### 1.1.1. Magnetic Separation Operation

In the dissolution and adsorption step S102, the external magnetic field acts on the magnetic bead 2 where the nucleic acid is adsorbed, and magnetic attraction occurs. Accordingly, the magnetic bead 2 is moved to an inner wall of the container 1 and fixed. As a result, as shown in FIG. 3, the magnetic bead 2 as the solid phase and the liquid 3 as the liquid phase can be separated. In the specification, such operation of fixing the magnetic bead 2 by applying an external magnetic field is referred to as a "magnetic separation operation".

Before the magnetic separation operation, the contents in the container 1 are stirred as necessary. Accordingly, a probability that the nucleic acid is adsorbed by the magnetic bead 2 increases. In the stirring, for example, a vortex mixer, hand shaking, or pipetting is used.

In the application of the external magnetic field, for example, a magnet 5 disposed beside the container is used. The magnet 5 may be an electromagnet or a permanent magnet. When the external magnetic field acts on the magnetic bead 2, the magnetic bead 2 moves toward the magnet 5.

After the magnetic separation operation, an acceleration may be applied to the container as necessary. Accordingly, the liquid 3 adhering to the magnetic bead 2 can be shaken off, and the unseparated liquid 3 can be reduced. The acceleration may be a centrifugal acceleration. A centrifugal separator may be used to apply the centrifugal acceleration.

### 1.1.2. Liquid Discharge Operation

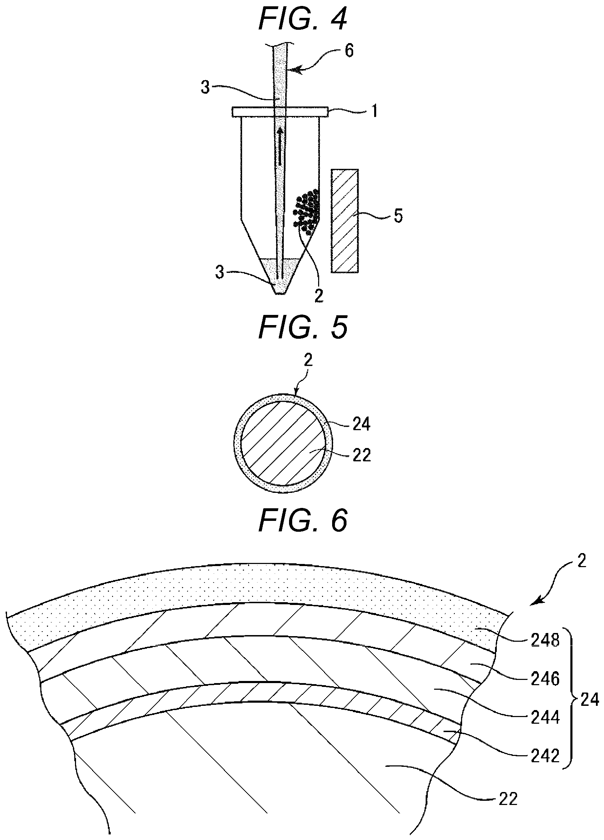

In the dissolution and adsorption step S102, in a state in which the magnetic bead 2 is fixed to the inner wall of the container 1, as shown in FIG. 4, the liquid 3 accumulated at a bottom of the container 1 is suctioned and discharged by, for example, a pipette 6. In the specification, such an operation of discharging the liquid 3 is referred to as a "liquid discharge operation". By the liquid discharge operation, the magnetic bead 2 where the nucleic acid is adsorbed remains in the container 1.

### 1.2. Washing Step

In the washing step S108, the magnetic bead 2 where the nucleic acid is adsorbed is washed. Washing refers to an operation of removing foreign substances by bringing the magnetic bead 2 where the nucleic acid is adsorbed into contact with a washing liquid and then separating the magnetic bead 2 again in order to remove the foreign substances adsorbed at the magnetic bead 2.

Specifically, after the washing liquid is charged into the container 1 in which the magnetic bead 2 where the nucleic acid is adsorbed is charged, the magnetic separation operation and the liquid discharge operation are performed again.

Among these, in the magnetic separation operation, first, the washing liquid is supplied into the container 1 by a pipette or the like. Then, the magnetic bead 2 and the washing liquid are stirred. Accordingly, the washing liquid comes into contact with the magnetic bead 2, and the magnetic bead 2 where the nucleic acid is adsorbed is washed. In the stirring, for example, a vortex mixer, hand shaking, or pipetting is used. At this time, the application of the external magnetic field may be temporarily turned off. Accordingly, since the magnetic beads 2 are redispersed in the washing liquid, washing efficiency can be further improved.

Next, as the liquid discharge operation, the washing liquid accumulated at the bottom of the container 1 is discharged in a state in which the magnetic bead 2 is fixed to the inner wall of the container 1. The magnetic bead 2 is washed by performing the above-described supply and discharge of the washing liquid at least once. Accordingly, the foreign substances can be removed with high accuracy.

The washing liquid is not particularly limited as long as the washing liquid is a liquid that does not promote elution of the nucleic acid and does not promote binding of the foreign substances to the magnetic bead 2, and examples thereof include organic solvents such as ethanol, isopropyl alcohol, and acetone, aqueous solutions thereof, and low-salt-concentration aqueous solutions.

The washing liquid may contain a surfactant such as Triton (registered trademark), Tween (registered trademark), or SDS. The washing liquid may contain a chaotropic substance such as guanidine hydrochloride.

The washing step S108 may be performed as necessary and may be omitted when washing is not necessary.

### 1.3. Elution Step

In the elution step S110, the nucleic acid adsorbed at the magnetic bead 2 is eluted into an elution liquid. The elution is an operation of transferring the nucleic acid to the elution liquid by bringing the magnetic bead 2 where the nucleic acid is adsorbed into contact with the elution liquid and then separating the magnetic bead 2 again.

Specifically, after the elution liquid is charged into the container 1 in which the magnetic bead 2 where the nucleic acid is adsorbed is charged, the magnetic separation operation and the liquid discharge operation are performed again.

Among these, in the magnetic separation operation, first, the elution liquid is supplied into the container 1 by a pipette or the like. Then, the magnetic bead 2 and the elution liquid are stirred. Accordingly, the elution liquid comes into contact with the magnetic bead 2, and the nucleic acid is eluted into the elution liquid. In the stirring, for example, a vortex mixer, hand shaking, or pipetting is used. At this time, the application of the external magnetic field may be temporarily turned off. Accordingly, since the magnetic beads 2 are dispersed in the elution liquid, elution efficiency can be further improved.

Next, as the liquid discharge operation, the elution liquid accumulated at the bottom of the container 1 is discharged in a state in which the magnetic bead 2 is fixed to the inner wall of the container 1. Accordingly, the elution liquid containing the nucleic acid can be collected.

The elution liquid is not particularly limited as long as the elution liquid is a liquid that promotes the elution of the nucleic acid from the magnetic bead 2 where the nucleic acid is adsorbed, and for example, in addition to water such as sterilized water or pure water, a TE buffer, that is, an aqueous solution which contains 10 mM Tris-HCl buffer and 1 mM EDTA and which has a pH of about 8 is preferably used.

The elution liquid may contain a surfactant such as Triton (registered trademark), Tween (registered trademark), or SDS. In addition, sodium azide may be contained as a preservative.

In the elution step S110, the elution liquid may be heated. Accordingly, the elution of the nucleic acid can be promoted. A heating temperature of the elution liquid is not particularly limited, and is preferably 70°C or higher and 200°C or lower, more preferably 80°C or higher and 150°C or lower, and still more preferably 95°C or higher and 125°C or lower.

### 2. Biological Material Extraction Magnetic Bead

Next, the magnetic bead 2 (biological material extraction magnetic bead according to the embodiment) will be described. The magnetic bead 2 is a particle that has magnetism and whose surface exhibits a binding affinity with a biological material.

FIG. 5 is a cross-sectional view showing the magnetic bead 2 according to the embodiment. The magnetic bead 2 shown in FIG. 5 includes a magnetic metal powder 22 and a coating film 24. A metal powder having magnetism is used as the magnetic metal powder 22. At least a surface of the coating film 24 is formed of a substance or a chemical structure having a binding affinity with the biological material. In the magnetic separation operation, a particle group that is an aggregate of such magnetic beads 2 is used. In the specification, the magnetic bead 2 refers to the particle group or one particle constituting the particle group.

A saturation magnetization of the magnetic bead 2 is preferably 50 emu/g or more, more preferably 80 emu/g or more, and still more preferably 100 emu/g or more. The saturation magnetization is a magnetization value in a case where a magnetization exhibited by a magnetic material when a sufficiently large magnetic field is externally applied is constant regardless of the magnetic field. When the saturation magnetization is within the above-described range, a function as a magnetic material can be sufficiently exhibited. Specifically, since a movement speed of the magnetic bead 2 in a magnetic field can be increased, a time required for magnetic separation can be shortened. The saturation magnetization of the magnetic bead 2 affects an adsorption force when the magnetic bead 2 is fixed by an external magnetic field. When the saturation magnetization is within the above-described range, a sufficiently high adsorption force can be obtained, and therefore, when the liquid 3 is discharged in a state in which the magnetic bead 2 is fixed, the magnetic bead 2 can be prevented from being discharged together with the liquid 3. Accordingly, it is possible to prevent a decrease in a nucleic acid yield due to a decrease in the number of the magnetic beads 2.

An upper limit value of the saturation magnetization of the magnetic bead 2 is not particularly limited, and is preferably 220 emu/g or less from the viewpoint of ease of selection of a material suitable for a balance between performance and cost.

The saturation magnetization of the magnetic bead 2 can be measured by a vibrating sample magnetometer (VSM) or the like. As the vibrating sample magnetometer, for example, TM-VSM1230-MHHL manufactured by Tamagawa Seisakusyo Co., Ltd. may be used. A maximum applied magnetic field when measuring the saturation magnetization is, for example, 0.5 T or more.

An average particle diameter D50 of the magnetic bead 2 is preferably 0.5 µm or more and 50 µm or less, more preferably 1 µm or more and 30 µm or less, still more preferably 2 µm or more and 20 µm or less, and particularly preferably 3 µm or more and 15 µm or less. When the average particle diameter D50 of the magnetic bead 2 is within the above-described range, a specific surface area of the magnetic bead 2 can be sufficiently large, and an attractive force and an adsorption force suitable for magnetic separation can be generated in the magnetic bead 2. In addition, aggregation of the magnetic beads 2 can be prevented, and dispersibility can be improved. When the average particle diameter D50 of the magnetic bead 2 is less than the lower limit value, a magnetization value of the magnetic bead 2 is small, the magnetic bead 2 easily aggregates, and as a result, nucleic acid extraction efficiency may decrease. In addition, the movement speed of the magnetic bead 2 may decrease, and a time required for magnetic separation may increase. On the other hand, when the average particle diameter D50 of the magnetic bead 2 exceeds the upper limit value, since the specific surface area of the magnetic bead 2 is small, a sufficient amount of the nucleic acid cannot be adsorbed, and an extraction amount of the nucleic acid may decrease. In addition, the magnetic bead 2 is likely to settle, the number of the magnetic beads 2 that can contribute to the extraction of the nucleic acid may decrease, and the nucleic acid extraction efficiency may decrease.

The average particle diameter D50 of the magnetic bead 2 can be obtained from a cumulative distribution curve obtained from a volume-based particle size distribution measured by a laser diffraction and dispersion method. Specifically, in the cumulative distribution curve, a particle diameter (median diameter) where a cumulative value is 50% from a small diameter side is the average particle diameter D50 of the magnetic bead 2. Examples of an apparatus for measuring the particle size distribution by the laser diffraction and dispersion method include MT3300 series manufactured by MicrotracBEL. The method is not limited to the laser diffraction and dispersion method, and a method such as image analysis may be used.

A 90% particle diameter of the magnetic bead 2 is defined as D90. In the magnetic bead 2, a ratio D90/D50 of the 90% particle diameter D90 to the average particle diameter D50 is preferably 3.00 or less, more preferably 2.00 or less, and still more preferably 1.75 or less. Accordingly, since a content of coarse particles is low, it is possible to prevent the coarse particles from attracting relatively small surrounding particles and aggregating to form an aggregate. When an aggregate is generated, the aggregate settles due to own weight, which may cause a decrease in extraction efficiency and an increase in an examination time of the biological material. Therefore, when the ratio D90/D50 is within the above-described range, occurrence of such a problem can be prevented. When the ratio D90/D50 exceeds the upper limit value, the content of coarse particles is high, and therefore, even when the application of the external magnetic field is turned off, the dispersibility of the magnetic bead 2 decreases, and aggregation may be likely to occur.

The 90% particle diameter D90 of the magnetic bead 2 can be obtained from a cumulative distribution curve obtained from a volume-based particle size distribution measured by a laser diffraction and dispersion method. Specifically, in the cumulative distribution curve, a particle diameter where a cumulative value is 90% from a small diameter side is the 90% particle diameter D90 of the magnetic bead 2.

A ratio t/D50 of t to D50, in which t is an average thickness of the coating film 24 and D50 is the average particle diameter of the magnetic bead 2, is preferably 0.0001 or more and 0.05 or less, and more preferably 0.001 or more and 0.01 or less. When t/D50 is less than the lower limit value, a ratio of the thickness of the coating film 24 to the size of the magnetic metal powder 22 is excessively small, and thus the coating film 24 may be broken or peeled off when the magnetic beads 2 collide with each other or the magnetic bead 2 collides with the inner wall of the container 1 or the like. Therefore, an amount of the nucleic acid adsorbed and extracted by the surface of the coating film 24 decreases, and the extraction efficiency may decrease. When there are fragments of the peeled coating film 24 or the magnetic metal powder 22 in an extracted liquid, the fragments may be mixed as a foreign substance (contamination) at the same time when the nucleic acid is extracted. Further, the magnetic metal powder 22 may be exposed due to breaking and peeling of the coating film 24, and elution of iron ions or the like may occur when the magnetic metal powder 22 comes into contact with an acidic solution or the like, resulting in a decrease in the nucleic acid extraction efficiency. On the other hand, when t/D50 exceeds the upper limit value, a volume ratio of the coating film 24 to an entire volume of the magnetic bead 2 is large, and a magnetization per volume of the magnetic bead 2 may decrease. Accordingly, the movement speed when the external magnetic field acts on the magnetic bead 2 decreases, and the time required for magnetic separation may increase.

The thickness of the coating film 24 can be measured from, for example, a cross-sectional observation image of the magnetic bead 2 observed by a transmission electron microscope or a scanning electron microscope. The average thickness t of the coating film 24 can be calculated by acquiring a plurality of observation images and averaging measured values from image processing or the like. For example, the average thickness t is a value obtained by measuring the thickness of the coating film 24 at five or more positions for one magnetic bead 2, obtaining an average value thereof, and then averaging the average value by ten or more magnetic beads 2. Furthermore, intensities of a Si-K characteristic X-ray and a Fe-L characteristic X-ray may be compared using an analysis apparatus such as an energy dispersive X-ray spectroscopy (EDX), and the thickness of the coating film 24 may be calculated based on a comparison result. That is, as will be described later, when the coating film 24 contains silicon and the magnetic metal powder 22 includes a Fe-based alloy, an intensity ratio of a Si-K characteristic X-ray derived from the coating film 24 to a sum of a Fe-L characteristic X-ray derived from the magnetic metal powder 22 and a Si-K characteristic X-ray derived from the coating film 24 can be converted into the thickness of the coating film 24.

A coercive force Hc of the magnetic bead 2 is preferably 1500 A/m or less, more preferably 800 A/m or less, still more preferably 400 A/m or less, and particularly preferably 100 A/m or less. The coercive force Hc refers to a value of an external magnetic field in an opposite direction required to return a magnetized magnetic material to an unmagnetized state. That is, the coercive force Hc means a resistance force against an external magnetic field. As the coercive force Hc of the magnetic bead 2 decreases, the magnetic beads 2 are less likely to aggregate even when being switched from a state in which a magnetic field is applied to a state in which no magnetic field is applied, and the magnetic beads 2 can be uniformly dispersed in a dispersion liquid. Further, even when the switching of magnetic field application is repeated, since redispersibility of the magnetic beads 2 is improved as the coercive force Hc decreases, aggregation of the magnetic beads 2 can be further prevented. A lower limit value of the coercive force Hc of the magnetic bead 2 is not particularly limited, and is preferably 5 A/m or more from the viewpoint of ease of selection of a material suitable for a balance between performance and cost.

The coercive force Hc of the magnetic bead can be measured by a vibrating sample magnetometer or the like in the same manner as the saturation magnetization described above. A maximum applied magnetic field when measuring the coercive force Hc is, for example, 15 kOe.

A relative permeability of the magnetic bead 2 is preferably 5 or more. When the relative permeability of the magnetic bead 2 is less than the lower limit value, the movement speed of the magnetic bead 2 decreases, and the time required for magnetic separation may be longer. An upper limit value of the relative permeability of the magnetic bead 2 is not particularly limited, and since the magnetic bead 2 is in a powder form, the relative permeability is usually substantially 100 or less due to an influence of a demagnetizing field.

### 2.1. Magnetic Metal Powder

The magnetic metal powder 22 is particles having magnetism, and preferably contains at least one of Fe, Co, and Ni as an element. In particular, from the viewpoint of obtaining a high saturation magnetization, a composition of the magnetic metal powder 22 is preferably an alloy containing Fe as a main component (Fe-based alloy). Specifically, an atomic ratio of Fe is more preferably 50% or more, and still more preferably 70% or more. Examples of the Fe-based alloy include a Fe-Co alloy, a Fe-Ni alloy, a Fe-Co-Ni alloy, and compounds containing Fe, Co, and Ni. From the viewpoint of obtaining a high magnetization, as the magnetic metal powder 22, a carbonyl iron powder made of substantially 100 mass% of Fe, a Fe-Si alloy powder, a Fe-Si-Cr alloy powder, or the like is preferably used. According to such a Fe-based alloy, it is possible to obtain the magnetic metal powder 22 having a high saturation magnetization and a high permeability even when a particle diameter is small. Accordingly, it is possible to obtain the magnetic bead 2 whose movement speed due to the action of the external magnetic field is high and whose attractive force when captured by the external magnetic field is large. As a result, the time required for magnetic separation can be shortened, and the magnetic bead 2 can be prevented from being mixed into the elution liquid and becoming a foreign substance.

The Fe-based alloy may contain one or two or more selected from the group consisting of Cr, Nb, Cu, Al, Mn, Mo, Si, Sn, B, C, P, Ti, and Zr according to target properties, in addition to an element exhibiting strong magnetism alone like Fe described above. Si is a main element in an alloy powder and is also an element that promotes amorphization.

The Fe-based alloy may contain an impurity as long as an effect of the magnetic metal powder 22 is not impaired. The impurity in the embodiment is an element that is unintentionally mixed during a raw material of the magnetic metal powder 22 or production of the magnetic bead 2. The impurity is not particularly limited, and examples thereof include O, N, S, Na, Mg, and K.

An example of the Fe-based alloy is an alloy having a Si content of preferably 1.0 atomic% or more and 30.0 atomic% or less, more preferably 1.5 atomic% or more and 13.0 atomic% or less, and still more preferably 2.0 atomic% or more and 7.0 atomic% or less. Since such an alloy has a high permeability, the saturation magnetization tends to be high.

The Fe-based alloy may contain at least one of boron (B) having a content of 5.0 atomic% or more and 16.0 atomic% or less and carbon (C) having a content of 0.5 atomic% or more and 5.0 atomic% or less. These elements are elements that promote amorphization, and contribute to forming a stable amorphous structure or a stable nanocrystal structure in the magnetic metal powder 22.

Further, the Fe-based alloy preferably contains chromium (Cr) having a content of 1.0 atomic% or more and 8.0 atomic% or less. Accordingly, corrosion resistance of the magnetic metal powder 22 can be improved.

A content of impurities is preferably 1.0 atomic% or less in total. At such a level, the effect of the magnetic metal powder 22 is not impaired even when impurities are contained.

A particularly preferable example of the Fe-based alloy is an alloy containing Fe as a main component, having a Si content of 2.0 mass% or more and 9.0 mass% or less, a B content of 1.0 mass% or more and 5.0 mass% or less, and a Cr content of 1.0 mass% or more and 3.0 mass% or less. Such a Fe-based alloy can have a stable amorphous structure, and thus has a low coercive force. Since the Fe content is high, the saturation magnetization is high. Further, since Cr is contained, corrosion resistance is improved, and elution of iron ions and the like can be prevented. Since iron ions may adversely influence a biological material examination, it is preferable to prevent elution of iron ions.

The elements and the composition of the magnetic metal powder 22 can be specified by an ICP emission analysis method defined in JIS G 1258:2014, a spark emission analysis method defined in JIS G 1253:2002, or the like. When the magnetic metal powder 22 is covered with the coating film 24, a measurement can be performed by the above-described methods after the coating film 24 is removed by a chemical or physical method. When it is difficult to remove the coating film 24, for example, analysis can be performed, after cutting the magnetic bead 2, on a portion of the magnetic metal powder 22 that is a core with an analysis apparatus such as an electron probe micro analyzer (EPMA) or an energy dispersive X-ray spectroscopy (EDX).

A Vickers hardness of the magnetic metal powder 22 is preferably 100 or more, more preferably 300 or more, and still more preferably 800 or more. A method of measuring the hardness of the magnetic metal powder 22 is, for example, as follows. A plurality of particles of the magnetic metal powder 22 are taken out, embedded in a resin to prepare a resin-embedded sample, and then a cross-section of the magnetic metal powder 22 is exposed at a surface of the resin-embedded sample through grinding and polishing. The cross-section is subjected to indentation with a micro Vickers tester, a nanoindenter, or the like, and a hardness is measured from a size of the indentation.

When the Vickers hardness of the magnetic metal powder 22 is less than the lower limit value, the magnetic metal powder 22 may be plastically deformed by an impact when the magnetic bead 2 undergoes collision. When plastic deformation occurs, the coating film 24 may be peeled off or detached. An upper limit value of the Vickers hardness is not particularly limited, and is preferably 3000 or less from the viewpoint of ease of selection of a material suitable for a balance of performance and cost.

A main metal structure constituting the magnetic metal powder 22 may take various forms such as a crystal structure, an amorphous structure, and a nanocrystal structure. An amorphous structure refers to a non-crystal structure in which no crystal is present, and a nanocrystal refers to a structure mainly formed of fine crystals having a crystal grain diameter of 100 nm or less. The amorphous structure and the nanocrystal structure impart a high hardness to the magnetic metal powder 22. When the structure is the amorphous structure or the nanocrystal structure, the coercive force Hc of the magnetic bead 2 has a particularly low value, which contributes to improvement in the redispersibility of the magnetic beads 2. A volume fraction of the magnetic metal powder 22 having an amorphous structure or a nanocrystal structure is preferably 40% or more, and more preferably 60% or more. The volume fraction is obtained from a result of crystal structure analysis by X-ray diffraction. Each of the crystal structure, the amorphous structure, and the nanocrystal structure may exist alone, or two or more thereof may co-exist.

The metal structure of the magnetic metal powder 22 can also be identified by the crystal structure analysis by an X-ray diffraction method on the magnetic metal powder 22. Further, the metal structure can be specified by analyzing a structure observation image or a diffraction pattern obtained with a transmission electron microscope (TEM) from a cut-out sample. For example, in the case of the amorphous structure, a diffraction peak derived from a metal crystal of an α-Fe phase or the like is not observed in peak analysis in the X-ray diffraction method. In the case of the amorphous structure, a so-called halo pattern is formed in an electron diffraction pattern by TEM, and formation of a spot due to a crystal is not observed. The nanocrystal structure is formed of a crystal structure having a grain size of, for example, 100 nm or less, and can be checked from a TEM observation image. More accurately, an average grain diameter can be calculated by image processing or the like from a plurality of TEM structure observation images in which there are a plurality of crystals. In addition, the crystal grain diameter can be estimated by a Sherer method based on a diffraction peak of a target crystal phase obtained by an X-ray diffraction method. Further, for a crystal structure having a large grain diameter, the crystal grain diameter can be measured by a method such as observing a cross-section with an optical microscope or a scanning electron microscope (SEM).

In order to obtain the amorphous structure and the nanocrystal structure, it is effective to increase a cooling rate at the time of cooling after a molten raw material is pulverized when producing the magnetic metal powder 22. Ease of forming the amorphous structure and the nanocrystal structure also depends on an alloy composition. As a specific alloy system suitable for forming the amorphous structure and the nanocrystal structure, a composition in which one or two or more selected from the group consisting of Cr, Si, B, C, P, Nb, and Cu are added to Fe is preferable.

The magnetic metal powder 22 is produced by a method according to a general metal powder production method. Examples of the production method include a melting process in which a metal is melted, solidified, and powdered, a chemical process in which a powder is produced by a reduction method or a carbonyl method, and a mechanical process in which a metal having a larger shape such as an ingot is mechanically pulverized to obtain a powder. Among these, the melting process is suitable for producing the magnetic metal powder 22.

Among production methods based on the melting process, a representative production method is an atomization method. According to such a method, a molten metal having a desired composition formed by melting is sprayed to form a powder.

The atomization method causes a molten metal to collide with a fluid (liquid or gas) injected at a high speed, be rapidly quenched and solidified to form a powder, and is classified into a water atomization method, a high-pressure water atomization method, a rotary water jet atomization method, a gas atomization method, and the like depending on a difference in a type of a cooling medium and an apparatus configuration. According to the atomization method, the magnetic metal powder 22 can be efficiently produced. Further, in the high-pressure water atomization method, the rotary waterjet atomization method, and the gas atomization method, a particle shape of the metal powder is closer to a spherical shape due to an action of surface tension.

### 2.2. Coating Film

As shown in FIG. 5, the coating film 24 covers a particle surface of the magnetic metal powder 22. The coating film 24 may be formed at at least a part of the particle surface of the magnetic metal powder 22, and preferably covers the entire particle surface.

FIG. 6 is a partially enlarged view of the coating film 24 shown in FIG. 5.

As shown in FIG. 6, the coating film 24 includes an inorganic oxide layer 242, a base layer 244, a gold layer 246, and an immobilization layer 248. That is, the coating film 24 has a multilayer structure including these layers. Each layer preferably covers an entire base thereof, but there may be a discontinuous portion.

### 2.2.1. Inorganic Oxide Layer

The inorganic oxide layer 242 covers the particle surface of the magnetic metal powder 22 and contains an inorganic oxide. Examples of the inorganic oxide include a silicon oxide, a magnesium oxide, a calcium oxide, an aluminum oxide, a titanium oxide, a zirconium oxide, a boron oxide, and an yttrium oxide, and one or a mixture of two or more thereof is used. Since the inorganic oxide layer 242 containing such an inorganic oxide is porous, a large contact area with the base layer 244 can be ensured. Accordingly, adhesion of the base layer 244 to the inorganic oxide layer 242 can be improved.

Among these, the inorganic oxide is preferably a silicon oxide or a titanium oxide. Since the silicon oxide and the titanium oxide are chemically stable, oxidation and corrosion of the magnetic metal powder 22 can be particularly prevented, and corrosion resistance of the magnetic bead 2 can be particularly improved.

The silicon oxide is represented by a composition formula of SiOₓ (0 < x ≤ 2), and is preferably SiO₂. The silicon oxide may form a composite oxide or a composite with one or two or more selected from the group consisting of Al, Ti, V, Nb, Cr, Mn, Sn, and Zr.

The titanium oxide is represented by a composition formula of TiOₓ (0 < x ≤ 2), and is preferably TiO₂. The titanium oxide may form a composite oxide or a composite with one or two or more selected from the group consisting of Si, Al, V, Nb, Cr, Mn, Sn, and Zr.

The inorganic oxide may contain a substance (impurity) other than the inorganic oxide within a range in which an effect thereof is not impaired, for example, at a ratio of 50 mass% or less of the inorganic oxide described above. When a silicon oxide is used as the inorganic oxide, examples of the impurity include C, N, and P. A composition of the inorganic oxide can be checked by, for example, EDX analysis or Auger electron spectroscopy.

The inorganic oxide layer 242 may cover a surface of one particle of the magnetic metal powder 22, or may cover a plurality of particles together.

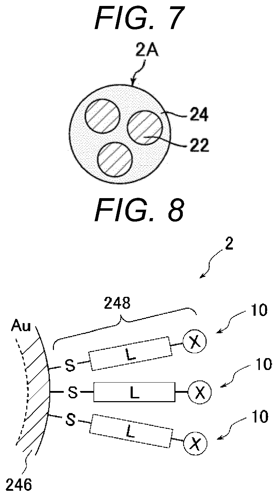

FIG. 7 is a cross-sectional view showing a modification of the magnetic bead 2 in FIG. 5.

A magnetic bead 2A shown in FIG. 7 contains a plurality of particles of the magnetic metal powder 22. The inorganic oxide layer 242 covers the plurality of particles. The base layer 244, the gold layer 246, and the immobilization layer 248 are also stacked on the inorganic oxide layer 242. In this way, the coating film 24 shown in FIG. 7 is formed. With such a magnetic bead 2A, the same effects as those of the magnetic bead 2 shown in FIG. 5 can still be obtained. The inorganic oxide layer 242 may cover each particle. The base layer 244, the gold layer 246, and the immobilization layer 248 may be provided across a plurality of particles. The base layer 244 may also cover each particle, and the gold layer 246 and the immobilization layer 248 may be provided across a plurality of particles.

The number of particles of the magnetic metal powder 22 of the magnetic bead 2A is not particularly limited, and is 2 or more and 100 or less.

An average thickness of the inorganic oxide layer 242 is preferably 10 nm or more and 200 nm or less, more preferably 20 nm or more and 150 nm or less, and still more preferably 30 nm or more and 100 nm or less. Accordingly, even when the magnetic beads 2 collide with each other or collide with the inner wall of the container or the like, the inorganic oxide layer 242 can be prevented from being broken or peeled off. As a result, elution of iron ions and the like due to exposure of the magnetic metal powder 22 can be prevented. A decrease in the magnetization per volume of the magnetic bead 2 can be prevented, and a decrease in the movement speed of the magnetic bead 2 can be prevented.

The average thickness of the inorganic oxide layer 242 is measured in the same manner as in the above-described method for measuring the average thickness of the coating film 24.

Examples of a method for forming the inorganic oxide layer 242 include a wet formation method such as a sol-gel method and a dry formation method such as a vapor-phase deposition method. Among these, a Stöber method, which is a type of the sol-gel method, or an atomic layer deposition (ALD) method can be preferably used. The Stöber method is a method of forming monodisperse particles by hydrolysis of a metal alkoxide. For example, when the inorganic oxide layer 242 is formed of a silicon oxide, the silicon oxide can be produced by a hydrolysis reaction of a silicon alkoxide. Before the inorganic oxide layer 242 is formed, a base thereof, for example, a surface of a particle of the magnetic metal powder 22 may be subjected to a washing treatment using water or an organic solvent.

### 2.2.2. Base Layer

The base layer 244 is a layer that can serve as a base of the gold layer 246 to be described later. The base of the gold layer 246 refers to a surface where gold can be favorably precipitated when the gold layer 246 is formed by an electroless gold plating method. Specifically, the base layer 244 contains a reducing agent for reducing gold ions to gold or a catalyst for a reduction reaction of reducing gold ions to gold. Such a base layer 244 improves adhesion of the gold layer 246. As a result, adhesion of the immobilization layer 248 formed at the gold layer 246 can be improved.

The reducing agent elutes into an electroless gold plating solution, ionizes, and releases electrons, thereby reducing gold ions to gold. Therefore, the base layer 244 containing the reducing agent enables formation of the gold layer 246 by a displacement plating method. Examples of the reducing agent include simple metals such as nickel, palladium, and copper, which exhibit a higher ionization tendency than gold, and alloys containing such metals.

When the reducing agent contained in the electroless gold plating solution causes a reduction reaction of gold ions, a catalyst has catalytic activity for catalyzing the reaction. Therefore, the base layer 244 containing a catalyst enables formation of the gold layer 246 by a self-catalyzed reduction plating method. Examples of the catalyst include simple metals such as nickel, palladium, and copper, and alloys containing such metals. A content of such metals in the base layer 244 is preferably more than 50 mass%, and more preferably 70 mass% or more.

Among these, the base layer 244 preferably contains nickel. Since the base layer 244 contains a simple substance of nickel or a nickel alloy, the gold layer 246 can be more favorably formed by an electroless gold plating method such as a displacement plating method or a self-catalyzed reduction plating method.

These reducing agents and catalysts may exist in any form. For example, the base layer 244 may be a plated film, a film formed by a vapor-phase deposition method, or a film formed by a liquid-phase deposition method. The plated film may be, for example, an electroless-plated film. Before the base layer 244 is formed, a base thereof, for example, a surface of the inorganic oxide layer 242 may be subjected to a surface treatment such as a degreasing treatment or an acid immersion treatment, or a washing treatment using water or an organic solvent.

An average thickness of the base layer 244 is not particularly limited, and is preferably 10 nm or more and 500 nm or less, more preferably 30 nm or more and 300 nm or less, and still more preferably 50 nm or more and 200 nm or less. Accordingly, it is possible to ensure a thickness for the base layer 244 to function sufficiently as a base.

The average thickness of the base layer 244 is measured in the same manner as in the above-described method for measuring the average thickness of the coating film 24.

A total average thickness of the inorganic oxide layer 242 and the base layer 244 is preferably 10 nm or more and 600 nm or less, more preferably 50 nm or more and 450 nm or less, and still more preferably 100 nm or more and 300 nm or less. Accordingly, the corrosion resistance of the magnetic bead 2 can be sufficiently ensured. In addition, a volume ratio of the coating film 24 to the entire volume of the magnetic bead 2 can be optimized, and a decrease in the magnetization per volume of the magnetic bead 2 can be prevented.

The average thickness of the base layer 244 may be smaller than the average thickness of the inorganic oxide layer 242, and is preferably set to be larger than the average thickness of the inorganic oxide layer 242. The average thickness of the base layer 244 is more preferably set to 1.2 times or more and 4.0 times or less the average thickness of the inorganic oxide layer 242, and still more preferably set to 1.5 times or more and 3.0 times or less. Accordingly, a function of the base layer 244 as a base, that is, a function of stably forming the gold layer 246, can be improved, and the corrosion resistance of the magnetic bead 2 can be improved. As a result, the immobilization layer 248 can be stabilized, oxidation and corrosion of the magnetic metal powder 22 can be prevented, and the magnetic bead 2 having particularly favorable nucleic acid extraction efficiency can be obtained.

The base layer 244 may have a multilayer structure. For example, a first layer may be a nickel-containing layer, and a second layer may be a palladium-containing layer. With such a multilayer structure, oxidation and corrosion of the base layer 244 can be more reliably prevented. As a result, the corrosion resistance of the magnetic bead 2 can be further improved.

### 2.2.3. Gold Layer

The gold layer 246 is a layer that can serve as a base of the immobilization layer 248. The base of the immobilization layer 248 refers to a surface where a compound contained in the immobilization layer 248 can be favorably bound. Specifically, examples of a constituent material of the gold layer 246 include a simple substance of gold and an alloy containing gold. Since the gold layer 246 has high corrosion resistance, a function of preventing oxidation and corrosion of the magnetic metal powder 22 is high. Accordingly, it is possible to prevent a decrease in the magnetization of the magnetic metal powder 22 and prevent a decrease in the nucleic acid extraction efficiency due to elution of iron ions or the like.

A gold content in the magnetic bead 2 is preferably 1 mass% or more and 30 mass% or less, more preferably 3 mass% or more and 25 mass% or less, and still more preferably 5 mass% or more and 20 mass% or less. Accordingly, coverage of the gold layer 246 can be sufficiently ensured, and the immobilization layer 248 can be formed evenly.

An average thickness of the gold layer 246 is preferably 0.5 nm or more and 50 nm or less, more preferably 1 nm or more and 30 nm or less, and still more preferably 2 nm or more and 20 nm or less. Accordingly, the corrosion resistance of the magnetic bead 2 can be particularly favorably ensured.

As described above, a method for forming the gold layer 246 may be, for example, an electroless gold plating method such as a displacement plating method or a self-catalyzed reduction plating method. Before the gold layer 246 is formed, a base thereof, for example, a surface of the base layer 244 may be subjected to a surface treatment such as a degreasing treatment or an acid immersion treatment, or a washing treatment using water or an organic solvent.

### 2.2.4. Immobilization Layer

The immobilization layer 248 contains a compound having a ligand or a ligand binding site. The ligand is a site having a function of specifically binding to a target biological material. The ligand binding site is a site (ligand reactive group) to which such a ligand can be bound. By including such an immobilization layer 248, the magnetic bead 2 can efficiently adsorb the target biological material.

Specific examples of the ligand include multidentate ligands such as nitrilotriacetic acid (NTA), iminodiacetic acid (IDA), phenanthroline, terpyridine, bipyridine, triethylenetetramine, tris(carboxymethyl)ethylenediamine, diethylenetriaminepentaacetic acid, poly(pyrazolyl)borate, 1,4,7-triazo-cyclononane, dimethylglyoxime, diphenylglyoxime, and derivatives thereof.

Among these, nitrilotriacetic acid (NTA) or iminodiacetic acid (IDA) is preferably used as the ligand.

Specific examples of the ligand binding site include an aldehyde group, a carboxy group, an amide group, N-hydroxysuccinimide (NHS), an amino group, a hydroxy group, -CH=CH-, and -(C=O)-CH=CH-.

Among these, an aldehyde group, a carboxy group, an amide group, or N-hydroxysuccinimide (NHS) is preferably used as the ligand binding site.

The ligand or the ligand binding site is bound to the gold layer 246 via a spacer provided as necessary and an Au-S bond. Since the Au-S bond is a strong bond, the Au-S bond contributes to strong immobilization of the ligand or the ligand binding site to the gold layer 246. Accordingly, even when various physical and chemical loads are applied to the magnetic bead 2 where the nucleic acid is adsorbed, it is possible to prevent detachment of the ligand. As a result, a decrease in efficiency of capturing the nucleic acid can be prevented, and the magnetic bead 2 that has favorable nucleic acid extraction efficiency can be obtained.

In the magnetic bead 2, the base layer 244 is provided as the base of the gold layer 246. The base layer 244 improves the adhesion of the gold layer 246. Accordingly, peeling of the gold layer 246 can be prevented, and from this viewpoint, the detachment of the ligand can also be prevented. Further, in the magnetic bead 2, the inorganic oxide layer 242 is provided as the base of the base layer 244. The inorganic oxide layer 242 improves the adhesion of the base layer 244. Accordingly, peeling of the base layer 244 can be prevented, and from this viewpoint, the detachment of the ligand can also be prevented. Therefore, the coating film 24 having the multilayer structure as described above can prevent the detachment of the ligand and implement the magnetic bead 2 having favorable nucleic acid extraction efficiency.

The compound having a ligand or a ligand binding site is a compound having a thiol group or a disulfide group that can form an Au-S bond, and is a reaction product of a reaction between a thiol derivative or a salt thereof to be described later and the gold layer 246.

FIG. 8 is a schematic diagram showing a state in which a compound 10 having a functional site X is bonded to the gold layer 246 via an Au-S bond. In FIG. 8, a ligand or a ligand binding site is referred to as the "functional site X".

The compound 10 shown in FIG. 8 includes an Au-S bond, a spacer L, and the functional site X.

The spacer L is a portion that couples a -S-bond to the functional site X. Examples of a unit structure constituting the spacer L include polyethylene glycol (PEG), polypropylene glycol (PPG), polybutylene glycol (PBG), a composite structure of a carbonate bond and PEG, a composite structure of an amide bond and PEG, and methylene, and a structure in which one or two or more of these structures co-exist.

As shown in FIG. 8, between adjacent compounds 10, self-organization is implemented by interaction between the spacers L, and the compounds 10 are easily aligned. Accordingly, it is possible to obtain the immobilization layer 248 in which the functional sites X are disposed at a high density. With such an immobilization layer 248, it is possible to increase a biological material adsorption amount per unit amount of the magnetic bead 2. The structure of the compound 10 shown in FIG. 8 may include a molecular chain branched structure (not shown).

Formula (1) below is an example of a structure of a thiol derivative that generates the compound 10.

R¹-(CH₂)ₓ-(C₂H₅O)_{y}-R² ... (1)

[In the formula (1), x is an integer of 2 or more and 18 or less, and y is an integer of 0 or more and 100 or less. In the formula (1), R¹ represents a thiol group or a disulfide bond, and R² represents the ligand or the ligand binding site.]

A thiol derivative having a structure represented by the formula (1) or a salt thereof can react with the gold layer 246 to produce the compound 10 in which the ligand or the ligand binding site can be disposed at a high density. Accordingly, the magnetic bead 2 having particularly favorable nucleic acid extraction efficiency can be obtained.

The structure represented by the formula (1) is all or a part of a molecular structure of the thiol derivative. Therefore, the molecular structure of the thiol derivative may include a structure represented by the formula (1) and another structure.

Examples of the salt of the thiol derivative include an alkali metal salt such as a lithium salt, a sodium salt, or a potassium salt, and an alkaline earth metal salt such as a magnesium salt or a calcium salt.

A thiol group is a reactive functional group represented by -SH. A disulfide bond is a functional group represented by -S-S- and is converted into a thiol group by reduction. Therefore, when R¹ in the formula (1) is a disulfide bond, the thiol derivative may have two sites where R¹ is removed from the formula (1). The thiol group reacts with gold to form a strong Au-S bond. A product obtained by such a reaction is the compound 10.

-(CH₂)ₓ-(C₂H₅O)_{y}- corresponds to the spacer L. Since the thiol derivative represented by the formula (1) contains methylene and PEG as the spacer L, the compound 10 is easily self-organized when bonded to the gold layer 246. Accordingly, the compounds 10 are aligned, and the functional sites X can be disposed at a high density.

In the formula (1), x representing the number of methylene is 2 or more and 18 or less, preferably 3 or more and 10 or less, and more preferably 4 or more and 6 or less. Accordingly, the compound 10 is stabilized, and deterioration or the like of the immobilization layer 248 due to the magnetic separation operation and the liquid discharge operation can be prevented. When x is less than the lower limit value, a length of the spacer L is small, and thus functionality of the functional site X may decrease, that is, the ligand or the ligand binding site may hardly function. When the length of the spacer L is small, it may be difficult to implement self-organization of the compound 10. On the other hand, when x exceeds the upper limit value, a molecular chain of the compound 10 may be excessively long, the compounds 10 may be difficult to be aligned, and the density of the functional sites X may not be sufficiently high.

In the formula (1), y representing the number of PEG is 0 or more and 100 or less, preferably 1 or more and 30 or less, and more preferably 2 or more and 10 or less. Accordingly, the compound 10 is stabilized. When y is less than the lower limit value, the length of the spacer L is small, and thus the functionality of the functional site X may decrease. When the length of the spacer L is small, it may be difficult to implement self-organization of the compound 10. On the other hand, when y exceeds the upper limit value, a molecular chain of the compound 10 may be excessively long, the compounds 10 may be difficult to be aligned, and the density of the functional sites X may not be sufficiently high.

Specific examples of the thiol derivative having a ligand include compounds represented by the following formulae (A-1) to (A-7).

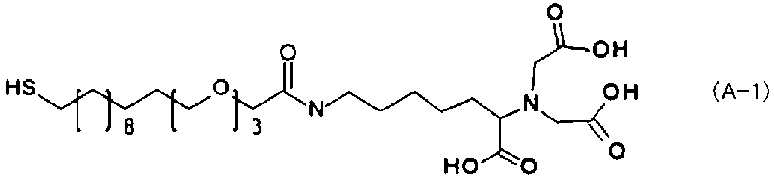

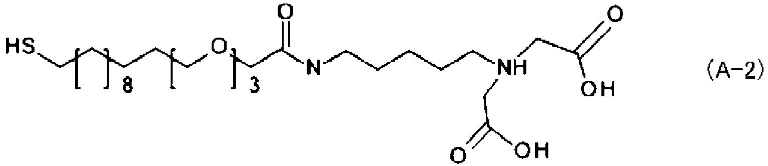

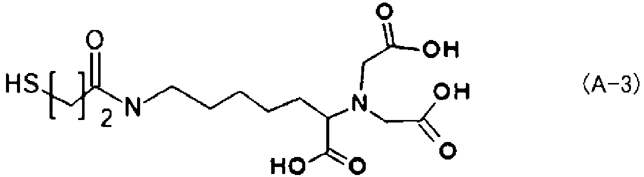

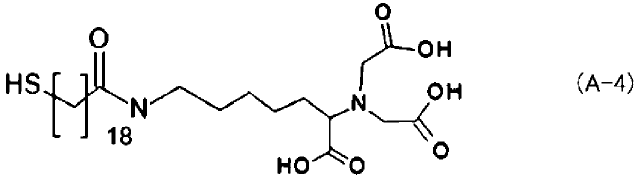

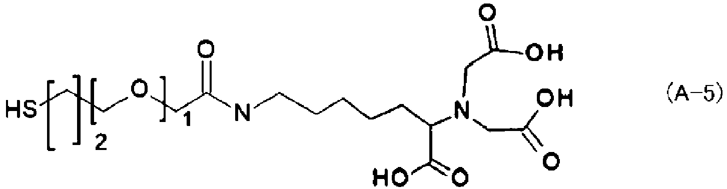

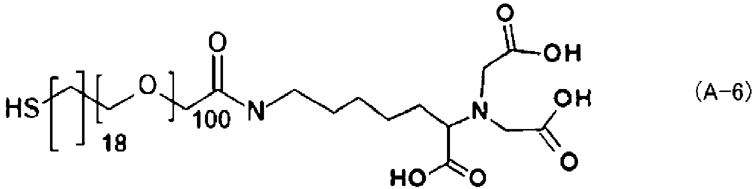

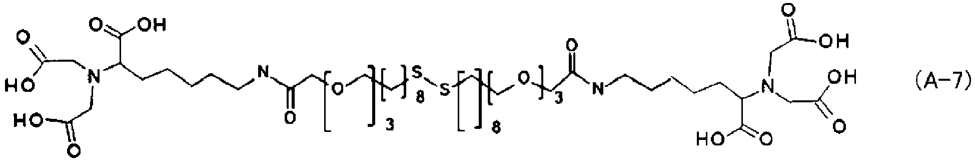

Specific examples of the thiol derivative having a ligand binding site include compounds represented by the following formulae (B-1) to (B-4).

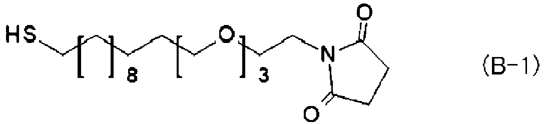

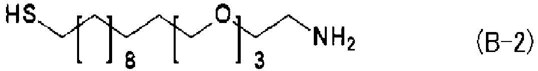

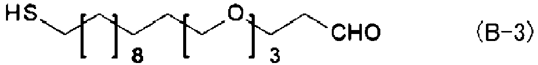

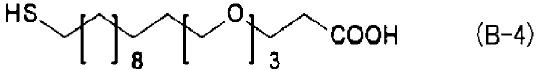

Such a compound is synthesized, for example, using a linker compound in which various functional groups are added to a PEG chain or an alkyl chain, by a method of binding such compounds via a PEG chain or an alkyl chain.

### 3. Effects of Embodiment

As described above, the magnetic bead 2 that is a biological material extraction magnetic bead according to the embodiment contains the magnetic metal powder 22, the inorganic oxide layer 242, the base layer 244, the gold layer 246, and the immobilization layer 248. The inorganic oxide layer 242 covers the particle surface of the magnetic metal powder 22 and contains an inorganic oxide. The base layer 244 covers the surface of the inorganic oxide layer 242 and contains a gold ion reducing agent or a catalyst for a gold ion reduction reaction. The gold layer 246 covers the surface of the base layer 244 and contains gold. The immobilization layer 248 contains the compound 10 that is bonded to the surface of the gold layer 246 via an Au-S bond and has a ligand or ligand binding site.

According to such a configuration, even when a load is applied, detachment of the ligand can be prevented, and oxidation and corrosion of the magnetic metal powder 22 can be prevented. Accordingly, the magnetic bead 2 having favorable biological material extraction efficiency can be obtained.

The compound 10 is preferably a reaction product of a thiol derivative represented by the following formula (1) or a salt thereof with the gold layer 246.

R¹-(CH₂)ₓ-(C₂H₅O)_{y}-R² ... (1)

[In the formula (1), x is an integer of 2 or more and 18 or less, and y is an integer of 0 or more and 100 or less. In the formula (1), R¹ represents a thiol group or a disulfide bond, and R² represents the ligand or the ligand binding site.]

A thiol derivative having a structure represented by the formula (1) or a salt thereof can react with the gold layer 246 to produce the compound 10 in which the ligand or the ligand binding site can be disposed at a high density. Accordingly, the magnetic bead 2 having particularly favorable biological material extraction efficiency can be obtained.

The inorganic oxide is preferably a silicon oxide or a titanium oxide.

Since the silicon oxide and the titanium oxide are chemically stable, oxidation and corrosion of the magnetic metal powder 22 can be particularly prevented, and corrosion resistance of the magnetic bead 2 can be particularly improved.

The base layer 244 preferably contains nickel.

Since the base layer 244 contains a simple substance of nickel or a nickel alloy, the gold layer 246 can be favorably formed by an electroless gold plating method such as a displacement plating method or a self-catalyzed reduction plating method.

The total average thickness of the inorganic oxide layer 242 and the base layer 244 is preferably 10 nm or more and 600 nm or less.

Accordingly, the corrosion resistance of the magnetic bead 2 can be sufficiently ensured. In addition, a volume ratio of the coating film 24 to the entire volume of the magnetic bead 2 can be optimized, and a decrease in the magnetization per volume of the magnetic bead 2 can be prevented.

The average particle diameter D50 of the magnetic bead 2 is preferably 0.5 µm or more and 50 µm or less.

Accordingly, the specific surface area of the magnetic bead 2 can be sufficiently large, and an attractive force and an adsorption force suitable for magnetic separation can be generated in the magnetic bead 2. In addition, aggregation of the magnetic beads 2 can be prevented, and dispersibility can be improved.

The saturation magnetization of the magnetic metal powder 22 is preferably 80 emu/g or more, and the coercive force of the magnetic metal powder 22 is preferably 100A/m or less.

Accordingly, since the movement speed of the magnetic bead 2 in the magnetic field can be increased, the time required for magnetic separation can be shortened. In addition, a sufficiently high adsorption force can be obtained, and therefore, when the liquid 3 is discharged in a state in which the magnetic bead 2 is fixed, the magnetic bead 2 can be prevented from being discharged together with the liquid 3. Accordingly, it is possible to prevent a decrease in a biological material yield due to a decrease in the number of the magnetic beads 2. Further, even when switching of magnetic field application is repeated, aggregation of the magnetic beads 2 can be prevented.

The average thickness of the base layer 244 is preferably 1.2 times or more and 4.0 times or less the average thickness of the inorganic oxide layer 242.

Accordingly, a function of the base layer 244 as a base, that is, a function of stably forming the gold layer 246, can be improved, and the corrosion resistance of the magnetic bead 2 can be improved. As a result, the immobilization layer 248 can be stabilized, oxidation and corrosion of the magnetic metal powder 22 can be prevented, and the magnetic bead 2 having particularly favorable biological material extraction efficiency can be obtained.

The biological material extraction magnetic bead according to the disclosure is described above based on the shown embodiment, but the disclosure is not limited thereto. For example, the biological material extraction magnetic bead according to the disclosure may be obtained by adding any component to the embodiment.

### Examples

Next, specific examples of the disclosure will be described.

### 4. Preparation of Magnetic Bead

### 4.1. Example 1

First, as a magnetic metal powder, a metal powder having an amorphous structure as a main structure was prepared, which was produced by a water atomization method. The prepared metal powder was washed with ultra-pure water and ethanol.

Next, a silicon oxide (SiOz) was formed into a film at a particle surface of the magnetic metal powder by a Stöber method to obtain an inorganic oxide layer. As a silicon alkoxide, tetraethoxysilane (TEOS) was used. The magnetic metal powder where the inorganic oxide layer was formed was subjected to a degreasing treatment and an acid immersion treatment.

Next, nickel was formed into a film at a surface of the inorganic oxide layer by an electroless nickel plating method to obtain a base layer. The magnetic metal powder where the base layer was formed was subjected to a degreasing treatment.

Next, gold was formed into a film at a surface of the base layer by a displacement type electroless gold plating method to obtain a gold layer. The magnetic metal powder where the gold layer was formed was washed with ultra-pure water and ethanol.

Next, an ethanol solution was prepared by dissolving a thiol derivative having a structure represented by a symbol shown in Table 1 in ethanol. Subsequently, the magnetic metal powder where the gold layer was formed was immersed in the ethanol solution and stirred. Accordingly, the thiol derivative was reacted with the gold layer to obtain an immobilization layer.

An average particle diameter, a saturation magnetization and a coercive force of the magnetic metal powder, and a coating film formation condition are as shown in Table 1. The symbol representing the thiol derivative shown in Table 1 corresponds to the formula (A-1) described above.

### 4.2. Examples 2 to 11

Magnetic beads were obtained in the same manner as in Example 1 except that a magnetic metal powder configuration and a coating film formation condition were as shown in Table 1. Symbols representing thiol derivatives shown in Table 1 correspond to thiol derivatives represented by the formulae (A-2) to (A-7) and the formulae (B-1) to (B-4) described above.

### 4.3. Comparative Example 1

Magnetic beads were obtained in the same manner as in Example 1 except that gold colloid particles were used instead of the magnetic metal powder. The gold colloid particles are particles containing only gold and do not include the inorganic oxide layer and the base layer.

### 4.4. Comparative Examples 2 and 3

Magnetic beads were obtained in the same manner as in Example 1, except that the immobilization layer was formed using a silane coupling agent instead of the thiol derivative. Symbols representing silane coupling agents shown in Table 1 correspond to silane coupling agents represented by the following formulae (C-1) and (D-1).

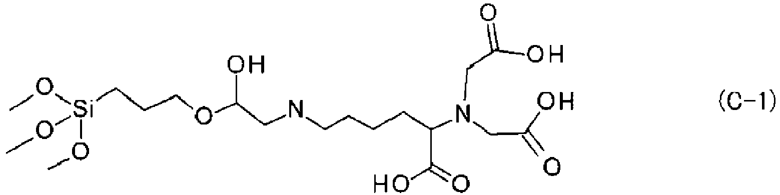

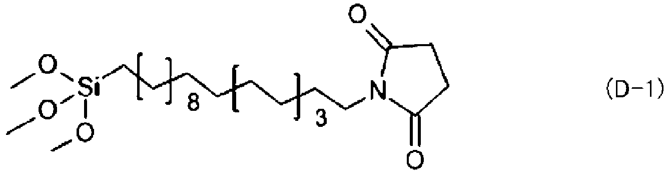

### 4.5. Examples 12 to 25

Magnetic beads were obtained in the same manner as in Example 1 except that a magnetic metal powder configuration and a coating film formation condition were as shown in Table 2. A symbol representing a thiol derivative shown in Table 2 corresponds to the formula (A-1) described above.

### 4.6. Comparative Example 4

Magnetic beads were obtained in the same manner as in Example 1 except that the inorganic oxide layer was omitted and a coating layer formation condition was as shown in Table 2.

### 4.7. Comparative Example 5

Magnetic beads were obtained in the same manner as in Example 1 except that the base layer was omitted and a coating layer formation condition was as shown in Table 2.

### 4.8. Comparative Example 6

Magnetic beads were obtained in the same manner as in Example 1 except that ferrite-containing particles were used instead of the magnetic metal powder, and a coating layer formation condition was as shown in Table 2. The ferrite-containing particles are particles obtained by dispersing ferrite particles in silica.

### 5. Evaluation of Magnetic Bead

### 5.1. Purification Yield of Biological Material

First, a protein A as a target biological material was dispersed in pure water to prepare a specimen aqueous solution in a microtube. A concentration of the protein A was 50 µg/mL.

Next, the magnetic beads of each of Examples and Comparative Examples were dispersed in the specimen aqueous solution such that a concentration was 30 mass%.

Next, a magnet was brought close to a side surface of the microtube, a magnetic field was applied, and then a supernatant was removed. Then, the magnetic beads remaining in the microtube were collected. Accordingly, the target biological material was adsorbed to the magnetic beads. Subsequently, the collected magnetic beads were dispersed in a PBS buffer aqueous solution to prepare a dispersion liquid. The PBS buffer aqueous solution was a phosphate buffer containing NaCl having a concentration of 137 mmol/L, Na₂HPO₄ having a concentration of 8.1 mmol/L, KCI having a concentration of 2.7 mmol/L, and KH₂PO₄ having a concentration of 1.5 mmol/L.

Next, the prepared dispersion liquid was stirred by inversion stirring for 30 minutes, followed by collection of a supernatant and addition of a BCA assay testing solution. Thereafter, an absorbance of the dispersion liquid to which the testing solution was added was measured by a UV-vis measuring instrument (U-3900H type spectrophotometer). Then, using a value of abs. -562 nm of an imidazole solution alone as a reference value, a ratio of a value of abs. -562 nm of the supernatant obtained using the magnetic beads of each of Examples and Comparative Examples was calculated. As the ratio is closer to 1.0, a purification yield of the biological material by the magnetic bead is larger. Subsequently, the calculated ratio was evaluated in view of the following evaluation criteria. Evaluation results are shown in Tables 1 and 2.
A: the ratio of the value of abs. -562 nm of the supernatant to the reference value is 0.8 or more
B: the ratio of the value of abs. -562 nm of the supernatant to the reference value is 0.7 or more and less than 0.8
C: the ratio of the value of abs. -562 nm of the supernatant to the reference value is 0.6 or more and less than 0.7
D: the ratio of the value of abs. -562 nm of the supernatant to the reference value is 0.3 or more and less than 0.6
E: the ratio of the value of abs. -562 nm of the supernatant to the reference value is less than 0.3

### 5.2. Redispersibility

First, a dispersion liquid was prepared by dispersing the magnetic beads of each of Examples and Comparative Examples in a PBS buffer aqueous solution to have a concentration of 30 mass%. The PBS buffer aqueous solution was the same as that used in 5.1.

Next, the prepared dispersion liquid was set in a laser diffraction and scattering particle diameter distribution measuring apparatus, and the average particle diameter D50 on a volume basis was measured. A measured value of the obtained average particle diameter D50 is taken as a value before separation of the target biological material, and is taken as a reference value.

Next, the magnetic beads were dispersed as in 5.1. in a specimen aqueous solution prepared in the same manner as in 5.1., and magnetic separation was performed to collect the magnetic beads where the biological material is adsorbed. Thereafter, as in 5.1., the collected magnetic beads were dispersed in a PBS buffer aqueous solution to prepare a dispersion liquid. Then, the average particle diameter D50 on a volume basis was measured in the same manner as described above, and a measured value thereof was set as a value after separation of the target biological material.

Next, a ratio of the value after separation to the reference value was calculated. As the ratio is closer to 1.0, redispersibility of the magnetic beads is more favorable. Subsequently, the calculated ratio was evaluated in view of the following evaluation criteria. Evaluation results are shown in Tables 1 and 2.
A: the ratio of the value after separation to the reference value is less than 1.1
B: the ratio of the value after separation to the reference value is 1.1 or more and less than 1.3
C: the ratio of the value after separation to the reference value is 1.3 or more and less than 1.5
D: the ratio of the value after separation to the reference value is 1.5 or more and less than 2.0
E: the ratio of the value after separation to the reference value is 2.0 or more

### 5.3. Corrosion Resistance

First, a dispersion liquid was prepared by dispersing the magnetic beads of each of Examples and Comparative Examples in a PBS buffer aqueous solution to have a concentration of 30 mass%. The PBS buffer aqueous solution was the same as that used in 5.1.

Next, the prepared dispersion liquid was allowed to stand for 10 days under conditions of an atmospheric pressure of 1013 hPa and a temperature of 70°C, and then an element concentration of a supernatant was measured using an ICP analyzer 5100 ICP-OES manufactured by Agilent Technologies, Inc.

Then, a difference from the element concentration of the supernatant was calculated using a value of an element concentration of the PBS buffer aqueous solution alone as a reference value. As a difference between an ion concentration of the supernatant and an ion concentration of the reference value is smaller, a magnetic component elution amount is smaller. Evaluation results are shown in Tables 1 and 2.
A: the difference in the ion concentration is less than 10 ppm
B: the difference in the ion concentration is 10 ppm or more and less than 20 ppm
C: the difference in the ion concentration is 20 ppm or more and less than 35 ppm
D: the difference in the ion concentration is 35 ppm or more and less than 100 ppm
E: the difference in the ion concentration is 100 ppm or more

### 5.4. Magnetic Separation Rate

First, a dispersion liquid was prepared by dispersing the magnetic beads of each of Examples and Comparative Examples in pure water at 25°C to have a concentration of 0.1 mass%. Next, the dispersion liquid was charged in a spectroscopic cell and stirred by ultrasonic irradiation or stirred by a vortex mixer. A stirring time was 1 minute. Next, the spectroscopic cell subjected to the stirring treatment was quickly set in a cell holder of a spectrophotometer. A magnet was attached to the cell holder in advance according to a position where the spectroscopic cell was disposed. A shortest distance between an outer wall of the spectroscopic cell set in the cell holder and the magnet was 2.0 mm, and a magnet having a surface magnetic flux density of 180 mT was used as the magnet.

Next, simultaneously with the start of standing of the spectroscopic cell, measurement of an absorbance at a wavelength of 550 nm in the spectroscopic cell was started. Then, a time until the measured absorbance was attenuated to 10% of an initial absorbance was measured, and a measurement result thereof was used as an evaluation index for evaluating a magnetic separation rate. The obtained evaluation index was evaluated in view of the following evaluation criteria. Evaluation results are shown in Tables 1 and 2.
A: the evaluation index of the magnetic separation rate is shorter than 20 seconds
B: the evaluation index of the magnetic separation rate is 20 seconds or longer and shorter than 30 seconds
C: the evaluation index of the magnetic separation rate is 30 seconds or longer and shorter than 45 seconds
D: the evaluation index of the magnetic separation rate is 45 seconds or longer and shorter than 60 seconds
E: the evaluation index of the magnetic separation rate is 60 seconds or longer

**Table 1**

| | | | | | Example 1 | Example 2 | Example 3 | Example 4 | Example 5 | Example 6 | Example 7 | Example 8 | Example 9 | Example 10 | Example 11 | Comparative Example 1 | Comparative Example 2 | Comparative Example 3 |
|---|---|---|---|---|---|---|---|---|---|---|---|---|---|---|---|---|---|---|
| Magnetic metal powder | | | Average particle diameter | µm | 3.2 | 3.2 | 3.2 | 3.2 | 3.2 | 3.2 | 3.2 | 3.2 | 3.2 | 3.2 | 3.2 | | 3.2 | 3.2 |
| | | | Saturation magnetization | emu/g | 108 | 108 | 108 | 108 | 108 | 108 | 108 | 108 | 108 | 108 | 108 | | 108 | 108 |
| | | | Coercive force | A/m | 36 | 36 | 36 | 36 | 36 | 36 | 36 | 36 | 36 | 36 | 36 | | 36 | 36 |
| Ferrite content | | | Average particle diameter | µm | | | | | | | | | | | | | | |
| | | | Saturation magnetization | emu/g | | | | | | | | | | | | | | |
| Gold colloid particles | | | Average particle diameter | µm | | | | | | | | | | | | 0.1 | | |
| Coating film formation condition | Inorganic oxide layer | Silicon oxide | Average thickness | nm | 50 | 50 | 50 | 50 | 50 | 50 | 50 | 50 | 50 | 50 | 50 | | 50 | 50 |
| | | Titanium oxide | Average thickness | nm | | | | | | | | | | | | | | |
| | Base layer | Nickel | Average thickness | nm | 100 | 100 | 100 | 100 | 100 | 100 | 100 | 100 | 100 | 100 | 100 | | 100 | 100 |
| | | Palladium | Average thickness | nm | | | | | | | | | | | | | | |
| | Total of inorganic oxide layer and base layer | | Average thickness | nm | 150 | 150 | 150 | 150 | 150 | 150 | 150 | 150 | 150 | 150 | 150 | 0 | 150 | 150 |
| | Gold layer | Gold | Content | mass% | 10 | 10 | 10 | 10 | 10 | 10 | 10 | 10 | 10 | 10 | 10 | 100 | 0 | 0 |
| | Immobilization layer | Thiol derivative having ligand | | - | A-1 | A-2 | A-3 | A-4 | A-5 | A-6 | A-7 | | | | | A-1 | | |
| | | Thiol derivative having ligand binding site | | - | | | | | | | | B-1 | B-2 | B-3 | B-4 | | | |
| | | Silane coupling agent having ligand | | - | | | | | | | | | | | | | C-1 | |
| | | Silane coupling agent having ligand binding site | | - | | | | | | | | | | | | | | D-1 |
| Evaluation result of magnetic bead | Purification yield | | | - | A | A | C | C | C | C | B | B | B | B | A | C | E | E |
| | Redispersibility | | | - | A | A | A | A | A | A | B | B | B | A | A | E | C | B |
| | Corrosion resistance | | | - | A | A | A | A | A | A | A | A | A | A | A | A | A | A |
| | Magnetic separation rate | | | - | A | A | A | A | A | A | A | A | A | A | A | E | A | A |

**Table 2**

| | | | | | Example 12 | Example 13 | Example 14 | Example 15 | Example 16 | Example 17 | Example 18 | Example 19 | Example 20 | Example 21 | Example 22 | Example 23 | Example 24 | Example 25 | Comparative Example 4 | Comparative Example 5 | Comparative Example 6 |
|---|---|---|---|---|---|---|---|---|---|---|---|---|---|---|---|---|---|---|---|---|---|
| Magnetic metal powder | | | Average particle diameter | µm | 3.2 | 3.2 | 3.2 | 3.2 | 3.2 | 3.2 | 1.1 | 10.5 | 45.7 | 3.2 | 3.2 | 3.2 | 3.2 | 3.2 | 3.2 | 3.2 | |
| | | | Saturation magnetization | emu/g | 108 | 108 | 108 | 108 | 108 | 108 | 93 | 113 | 114 | 108 | 108 | 108 | 108 | 108 | 108 | 108 | |
| | | | Coercive force | A/m | 36 | 36 | 36 | 36 | 36 | 36 | 28 | 48 | 89 | 36 | 36 | 36 | 36 | 36 | 36 | 36 | |
| Ferrite content | | | Average particle diameter | µm | | | | | | | | | | | | | | | | | 2.8 |
| | | | Saturation magnetization | emu/g | | | | | | | | | | | | | | | | | 11 |
| Gold colloid particles | | | Average particle diameter | µm | | | | | | | | | | | | | | | | | |
| Coating film formation condition | Inorganic oxide layer | Silicon oxide | Average thickness | nm | 10 | | 15 | 50 | 150 | 150 | 50 | 50 | 50 | 50 | 50 | 50 | 5 | 200 | | 150 | 50 |
| | | Titanium oxide | Average thickness | nm | | 50 | | | | | | | | | | | | | | | |
| | Base layer | Nickel | Average thickness | nm | 100 | 100 | 15 | 150 | 250 | 450 | 15 | 100 | 100 | | 100 | 100 | 15 | 450 | 400 | | 100 |
| | | Palladium | Average thickness | nm | | | | | | | | | | 100 | | | | | | | |
| | Total of inorganic oxide layer and base layer | | Average thickness | nm | 110 | 150 | 30 | 200 | 400 | 600 | 65 | 150 | 150 | 150 | 150 | 150 | 20 | 650 | 400 | 150 | 150 |
| | Gold layer | Gold | Content | mass% | 10 | 10 | 10 | 10 | 10 | 10 | 10 | 10 | 10 | 10 | 5 | 20 | 10 | 10 | 10 | 10 | 10 |
| | Immobilization layer | Thiol derivative having ligand | | - | A-1 | A-1 | A-1 | A-1 | A-1 | A-1 | A-1 | A-1 | A-1 | A-1 | A-1 | A-1 | A-1 | A-1 | A-1 | A-1 | A-1 |
| | | Thiol derivative having ligand binding site | | - | | | | | | | | | | | | | | | | | |
| | | Silane coupling agent having ligand | | - | | | | | | | | | | | | | | | | | |
| | | Silane coupling agent having ligand binding site | | - | | | | | | | | | | | | | | | | | |
| Evaluation result of magnetic bead | Purification yield | | | - | A | A | B | B | B | C | C | A | B | B | A | A | C | C | E | E | D |
| | Redispersibility | | | - | B | A | A | B | A | C | C | B | B | B | B | B | A | C | E | E | A |
| | Corrosion resistance | | | - | A | A | C | B | A | A | B | A | A | B | 8 | A | C | A | E | A | C |
| | Magnetic separation rate | | | - | B | A | A | A | A | B | B | A | A | A | A | A | A | B | B | B | D |

As shown in Tables 1 and 2, in the case of the magnetic beads of each Example, the purification yield of the biological material is large and the redispersibility is also favorable even after the magnetic separation operation. The magnetic beads of each Example have favorable corrosion resistance even in a state of being immersed in the PBS buffer aqueous solution containing an acid for 10 days. Further, it was also found that the magnetic beads of each Example have a sufficiently high magnetic separation rate.

From the above results, it is found that a magnetic bead having favorable biological material extraction efficiency can be provided according to the disclosure.

## Claims

1. A biological material extraction magnetic bead comprising:
a magnetic metal powder;
an inorganic oxide layer that covers a particle surface of the magnetic metal powder and contains an inorganic oxide;
a base layer that covers a surface of the inorganic oxide layer and contains a gold ion reducing agent or a catalyst for a gold ion reduction reaction;
a gold layer that covers a surface of the base layer and contains gold; and
an immobilization layer that is bonded to a surface of the gold layer via an Au-S bond and contains a compound having a ligand or a ligand binding site.

2. The biological material extraction magnetic bead according to claim 1, wherein
the compound is a reaction product of a thiol derivative represented by a following formula (1) or a salt thereof with the gold layer:
R¹-(CH₂)ₓ-(C₂H₅O)_{y}-R² ... (1)
[In the formula (1), x is an integer of 2 or more and 18 or less, and y is an integer of 0 or more and 100 or less. In the formula (1), R¹ represents a thiol group or a disulfide bond, and R² represents the ligand or the ligand binding site.]

3. The biological material extraction magnetic bead according to claim 1, wherein
the inorganic oxide is a silicon oxide or a titanium oxide.

4. The biological material extraction magnetic bead according to claim 1, wherein
the base layer contains nickel.

5. The biological material extraction magnetic bead according to claim 1, wherein
an average thickness of a total of the inorganic oxide layer and the base layer is 10 nm or more and 600 nm or less.

6. The biological material extraction magnetic bead according to claim 1, wherein
an average particle diameter is 0.5 µm or more and 50 µm or less.

7. The biological material extraction magnetic bead according to claim 1, wherein
a saturation magnetization of the magnetic metal powder is 80 emu/g or more, and
a coercive force of the magnetic metal powder is 100 A/m or less.

8. The biological material extraction magnetic bead according to claim 1, wherein
an average thickness of the base layer is 1.2 times or more and 4.0 times or less an average thickness of the inorganic oxide layer.
